# EUROPEAN PATENT APPLICATION

(11) **EP 4 691 408 A1**
(43) Date of publication of application: **11.02.2026**
(21) Application number: 24193131.0
(22) Date of filing: 06.08.2024
(51) Int. Cl.: A61B 90/00

(54) **MEDICAL VIEWING DEVICES AND METHODS TO OPERATE MEDICAL VIEWING DEVICES**

(71) Applicant: Leica Instruments (Singapore) Pte. Ltd., Singapore 618299 (SG)
(72) Inventor: Themelis, George, 618299 Singapore (SG)
(74) Representative: Paustian & Partner Patentanwälte mbB

(57) **Abstract**

A first aspect of the present disclosure is related to a medical viewing device, configured to:
- be worn by a human user, in particular on a user's head;
- obtain visual information about an environment from a medical observation means;
- obtain visual information about the environment from a camera;
- display to the user the environment in two or more different modes ;
wherein in an observation mode the viewing device provides a view from the medical observation means; and
wherein in a first awareness mode, the viewing device provides a view from the camera.

## Description

### Technical Field

This disclosure is related to medical viewing devices and methods for operating medical viewing devices.

### Background

Medical viewing devices such as surgical loupes are specialized eyewear designed to assist surgeons or other medical personnel by providing magnification and enhancing visual precision during various medical procedures. These sophisticated glasses e.g. play a crucial role in enabling surgeons to see small details with greater clarity, thus improving the accuracy and outcomes of surgeries. One of the notable features of surgical loupes is their ability to fold upwards, allowing the surgeon to easily switch between magnified and natural vision as needed. This functionality is particularly beneficial during operations, as it provides flexibility and convenience. When the magnification is required again, the loupes can be effortlessly folded back into position in front of the surgeon's eyes. This transition between magnified and natural vision helps maintain focus and efficiency in the operating room. Overall, such medical viewing devices are important tools that contribute significantly to the success and precision of modern medical practices. Therefore, further improvements of such medical viewing devices are desired.

### Summary

An object of the present disclosure is to improve a visual perception of a medical environment.

This object is solved by the disclosed embodiments, which are defined in particular by the subject matter of the independent claims. The dependent claims provide information for further embodiments. Various aspects and embodiments of these aspects are also disclosed in the summary and description below, which provide additional features and advantages.

A first aspect of the present disclosure is related to a medical viewing device, configured to:
- be worn by a human user, in particular on a user's head;
- obtain visual information about an environment from a medical observation means;
- obtain visual information about the environment from a camera;
- display to the user the environment in two or more different modes ; wherein in an observation mode the viewing device provides a view from the medical observation means; and
wherein in a first awareness mode, the viewing device provides a view from the camera.

A medical viewing device may for example comprise one or multiple screens or displays, in particular high-resolution screens or displays. Furthermore, a medical viewing device may comprise mounting means, for example a headband or temples, in order to enable a user to wear said medical device. A medical viewing device may also provide a three dimensional (3D) viewing experience for a user, for example, with an individual screen for each eye of the user. A medical viewing device may for example comprise a virtual reality (VR) or augmented reality (AR) headset.

A virtual reality (VR) headset may for example be a headset used to create an immersive digital environment that isolates users from the real world. Thus, a VR-Headset may comprise head-mounted displays with stereoscopic screens and motion sensors to simulate a 3D space. An augmented reality (AR) headset may for example overlay digital information onto the real world through transparent lenses or screens. Insofar, an augmented reality headset may use sensors and cameras to integrate digital content with the user's physical surroundings.

A medical observation means is configured to observe a medical relevant information, in particular an organ within a patient's body during a surgery or a medical examination, e.g. a prostate, a colon, or an eye. Therefore, the medical observation means can be positioned for optimal observation.

A medical observation means may be a digital camera, for example a camera of a microscope or a camera mounted on a medical device. For example, an endoscope may be equipped with a camera, in particular a high-definition camera, to allow a surgeon to view internal organs and tissues. In another example, surgical microscopes or operating microscopes may comprise integrated cameras to provide magnified, high-resolution images of the operating field, enabling more accurate interventions. In another example, a camera may be mounted on laparoscopic instruments offering detailed views of the operating field, e.g. of an abdominal cavity. Thus, a medical observation means may be an external device.

A camera may also be mounted on the medical viewing device in order to gather visual information from the environment surrounding the medical viewing device. If for example the medical viewing device is mounted on a user's head, such a camera my gather visual information, in other words images and image streams, of the user's environment. A camera may be configured to gather a 3D image or image stream of its environment and/ or may be configured to 360°-view of its environment. A camera may also comprise a fisheye-lens.

Obtaining visual information may comprise different actions of obtaining said information, for example fetching, receiving, determining.

An environment may be a three-dimensional (3D) space or room, for example an operating room, like for example the room surrounding a patient during a surgical operation. An environment may also be an operating field, e.g. an operating field within a patient, such as an abdominal cavity or parts of an intestine.

The visual information obtained by the medical observation means and by the camera about the environment may not be related to the same part of the environment. For example, an endoscope (medical observation means) may be configured to observe the inner part of a colon, while the camera may be configured to observe the patient (who is examined with the endoscope) as she/he is lying on the examination table.

Visual information may comprise data, in particular images or multiple images, also called image streams or video. The medical viewing device may thus be configured to receive data, in particular image streams, from the medical observation means and/ or the camera. For example, a medical viewing device may receive data wirelessly from one or multiple medical observations devices or cameras. It is also possible, that a medical viewing device may receive such data via one or multiple digital or optical cables or wires. Visual information from an environment may thus be image streams from an operating room or an operating field within a patient, depending on the medical device or respectively the camera that is transmitting the visual information to the medical viewing device.

A view may also be considered an image stream, in particular a real time image stream, from a camera or a medical observation means of an environment. In other words, by obtaining visual information about an environment and providing a view from said camera or a medical observation means, the medical viewing device may provide the user with both a real-time image stream, or view, from the operating field through the medical observation means and also a real-time image stream, or view, from the room surrounding the user, and possibly also the patient, through the camera.

Displaying the environment to the user in two or more different modes may be carried out in different ways. For example, the medical viewing device may display only one mode at a time, for example spanning the whole screen of the medical viewing device and/ or the whole visual field of the user. Thus, a user has a complete and unobstructed view of the view provided by this, "current", mode. A user may switch in between modes, for example using gestures, eye movement, voice commands or other forms of physical interaction with the medical viewing device such as pressing a button.

Displaying the environment to the user in two or more different modes may also be carried out by displaying said modes simultaneously. For example, the screen of the medical viewing device, or the visual field of the user, may be split in two or more areas so that each area may display the view provided by one mode. Said areas may also overlap or one area may be comprised in another. Insofar, a "window in window" representation is also possible, where one mode is displayed in a small area comprised in a larger area displaying another mode.

In an observation mode, the medical viewing device provides a view from the medical observation means. Thus, the medical viewing device may for example provide a view from an endoscope or a surgical microscope. In the observation mode, the user may thus view the operational field, for example the environment within the patient's body where a medical procedure is to be carried out. The view of the environment provided in an observation mode may also be enhanced, for example magnified, so that the user is provided with an ideal view of the environment.

In a first awareness mode, the medical viewing device provides a view from the camera. Thus, the medical viewing device may provide a view from for example an external camera filming the environment of the user. Such a camera may for example be mounted on the medical viewing device or may also be integrated in the medical viewing device. In a first awareness mode, the user may thus be provided with a view of their direct surroundings, such as the room they are situated in. Where an observation mode may comprise a magnified view that may be called "zoomed in", an awareness mode may comprise a view that may be considered "zoomed out".

A medical observation means may be configured such to provide the user, in particular a surgeon, with multiple views relevant to the procedure, in particular of surgical operations. In so far, the medical observation means can provide the user with both a view from the operating field within the patient that may be enhanced, e.g. magnified, and also an unobstructed view of the user's direct surroundings. Thus, the user does not need to remove or move the optical viewing device and consequently does not need to take their hands away from the procedure and in particular other surgical instruments.

An embodiment of the first aspect is related to a medical viewing device, comprising one or more cameras.

One or more cameras may for example be integrated into the medical viewing device and gather visual information of the environment surrounding the medical viewing device and thus surrounding the user. For example, a camera may be integrated into the medical viewing device so that it may gather visual information from the environment directly in front of the user. Additionally, or alternatively, a camera may be oriented such that it may gather visual information from the environment of the user's environment outside of his natural view. Insofar, cameras oriented in such a way may gather visual information of the whole environment surrounding the user, for example of the user's sides or back. A camera may for example be configured as a 360 ° camera or comprise a fisheye-lens in order to gather visual information.

Multiple cameras may serve the purpose of providing different perspectives of the environment, which may be used to build a larger view in one mode, or which may be used for different modes. For example, multiple camera perspectives may be digitally combined using image stitching techniques, which align and merge overlapping fields of view from different cameras to create a seamless, larger composite image. Such a process may involve algorithms to correct for differences in angle, perspective, and lighting, resulting in a comprehensive and continuous, for example panoramic, view.

Advantageously in such a configuration, no external input for the medical viewing device is required. Furthermore, multiple cameras may provide a more complete and unobstructed view of the environment.

An embodiment of the first aspect is related to a medical viewing device, configured to:
- obtain information from one or more external cameras.

An external camera may be a camera that is not integrated in the medical viewing device. An external camera may in particular be a camera that is not mounted on the medical viewing device, but it positioned elsewhere for example on the user or in the operating environment. Such a camera may for example be mounted to a ceiling of the room surrounding a patient or close to a light source.

Such a camera may advantageously enable a view of further perspectives that may not be achieved with a camera integrated in the medical viewing device. In particular, such a camera may enable perspectives that differ from the user's perspective, for example a bird's-eye-perspective.

Also advantageously, such a camera may provide an improved, possibly magnified view of a particular perspective, for example a different angle of the patient.

An embodiment of the first aspect is related to a medical viewing device, wherein in the first awareness mode the viewing device provides a view which is less magnified than in the observation mode.

Thus, in the first awareness mode, the users may be provided with a view that can be considered "zoomed out" in comparison to the observation mode. For example, such a view may be captured with a camera comprising a focal length of 50 mm, in particular paired with a full frame sensor, which is comparable with the field of view of a human eye.

In other words, the view the user may be provided with in the first observation mode may be considered a natural view of the user's environment, which advantageously allows for improved coordination within said environment. In particular, the view provided in the first awareness mode may stem from an integrated camera or a camera mounted on the medical viewing device capturing the user's perspective and moving with the movement of the user's head.

An embodiment of the first aspect is related to a medical viewing device, wherein in a second awareness mode, the viewing device provides a view which is less magnified than in the first awareness mode.

Thus, in the second awareness mode, the users may be provided with a view that can be considered "zoomed out" in comparison to the first awareness mode. In other words, the user may be provided with a view that is larger than their natural field of view. Such a view may for example comprise a panoramic view of the surroundings or environment of the user. As explained above, such a panoramic view may for example be captured with a fish-eye lens, a 180 ° or 360 ° camera or multiple cameras. Such a view may also be constructed digitally using aforementioned image stitching techniques.

Advantageously, a user may thus be provided with a view that extends his natural field of view, providing a better understanding of their surroundings. Insofar, a user may be enabled to view parts of their environment that are outside of their natural field of view without the necessity of turning their head.

An embodiment of the first aspect is related to a medical viewing device, configured to:
- switch between modes based on one or more of:
   -- a position-based information of the viewing device;
   -- an information in an image obtained by the viewing device;
   -- an information from an external sensor that observes a position and/or a motion of the medical viewing device.

A position-based information may refer to the position and/ or the angle or orientation of the medical viewing device. Such information may for example be obtained by a gyroscope. For example, a medical viewing device mounted on a user's head may use a gyroscope to obtain position-based information by measuring the rate of rotation around multiple axes, allowing it to track a user's head movements in real-time. Position-based information may thus comprise the orientation and movement of the medical viewing device in three-dimensional space, such as pitch, yaw, and roll. Insofar, a position-based information may indicate for example when a user moves their head away from the patient. In one example, a switch between modes may be carried out where the observation mode was provided as long as the user's head was directed towards the patient and the first or second awareness mode may be provided as soon as the user turned their head away from the patient. In one example, a switch between the first and second awareness mode may be carried out based on how far a user has turned their head away from the patient.

An information in an image obtained by the viewing device may for example be obtained with image recognition techniques. Image recognition techniques may involve the use of algorithms and machine learning to identify and classify objects, patterns, or features within digital images. For example, a patient may be recognized by analyzing facial features, body shape, and contextual cues. Thus, a mode switch may be carried out when a patient is no longer recognized on the view or image provided by a camera of the medical viewing device.

Image-based information may also come from an external sensor. Such a sensor may for example be configured as a stereotactic camera. A stereotactic camera may provide precise 3D localization of the medical viewing device, the user's head or the user's hands by capturing and analyzing multiple images from different angles. Based on for example the position of the user's head captured by such a camera, a switch between modes may be carried out.

Obtaining image-based information may comprise both determining the information by an internal sensor or receiving the information from an external sensor.

Insofar, a switching between modes and thus between views provided to the user may be based on natural interactions of the user with their environment. No additional gestures or actions are required that only serve the purpose of switching the modes. Switching modes thus becomes seamlessly integrated in the user's work and requires no additional action. In particular, the hands of the user remain free and do not have to be removed from the task at hand.

An embodiment of the first aspect is related to a medical viewing device, configured to: - switch between modes based on a gesture by the user, in particular by:
-- a head-based gesture;
-- a hand-based gesture.

A gesture may be recognized or identified based on one or more of the aforementioned types of information, in particular on a position-based information, an information in an image or an information from an external sensor. For example, a head-based gesture may be recognized based on the orientation and movement of the medical viewing device in three-dimensional space, such as pitch, yaw, and roll. Insofar, a head-based gesture may for example be a user moving their head away from the patient. In one example, a switch between modes may be carried out where the observation mode was provided as long as the user's head was directed towards the patient and the first or second awareness mode may be provided as soon as the user turned their head away from the patient. In one example, a switch between the first and second awareness mode may be carried out based on how far a user has turned their head away from the patient.

A head-based gesture may also comprise movement of the eye. Insofar, the medical viewing device may for example comprises sensors configured to scan or track the user's iris and to recognize eye movements or gestures. Eye movement or gesture recognition may interpret movements of the eyes to control for example the medical viewing device, in particular to input commands.

A hand-based gesture may be a movement of a user's hand, a specific change of position relative to the user or the user's surroundings as well as sings or symbols that are shown or carried out with a user's hand.

Insofar, a switching between modes and thus between views provided to the user may be based on interactions of the user with their environment and are not based on touch. In an environment like an operating room, hygiene is of great importance and reducing the necessity for a surgeon's physical touch is thus beneficial to the general hygiene.

An embodiment of the first aspect is related to a medical viewing device, configured to:
- switch between modes based on a gesture by the user, such that the viewing device switches to the observation mode if the head of the user moves slower than a pre-defined threshold.

For example, in an operating environment and while using a medical observation device, high focus and concentration as well as small and deliberate movement are required by a user of the medical viewing device, for example a surgeon. Thus, when the user's head moves slowly, the probability is very high, that the user is focusing on a specific object or environment. While focusing on a specific object or environment, it is beneficial to provide the user with the observation mode, thus providing him with a view from the medical observation device. By configuring the medical viewing device in such a way, the user may automatically be provided with the ideal mode with respect to the medical procedure which the user is focusing on.

An embodiment of the first aspect is related to a medical viewing device, configured to:
- switch between modes based on a gesture by the user, such that the viewing device switches to the first or the second awareness mode if the head of the user moves faster than a pre-defined threshold.

Contrary to the slower head movement during focus, a fast movement of a user's head may indicate an interruption of the medical procedure, for example if the user is turning his head to another person or towards other medical tools or machinery. It is thus beneficial, to provide the user with the first or second awareness mode, so that the user may be provided with an unobstructed view of their surroundings. By configuring the medical viewing device in such a way, the user may automatically be provided with the ideal mode with respect to the interruption, or end, of the medical procedure which the user is focusing on.

An embodiment of the first aspect is related to a medical viewing device, configured to:
- switch between modes based on a gesture by the user, such that the viewing device switches to the observation mode or to the first or the second awareness mode if the user performs a gesture based on tilting the user's head.

Tilting of the user's head may be recognized or identified based on one or more of the aforementioned types of information, in particular on a position-based information. For example, a head-based gesture, in particular a tilt, may be recognized based on the orientation and movement of the medical viewing device in three-dimensional space.

In a natural movement, a user may for example tilt their head downwards in order to get a view of the patient and thus of the medical procedure the user is carrying out. Thus, the medical viewing device may recognize this tilt downwards and provide the user with the observation mode, as a tilt downwards may indicate a user's desire to look at the procedure or his hands. The opposite may for example apply for a tilt of the head upwards, which may indicate a user's desire to look at something in his environment, for example a colleague. If the user tilts or lifts his head upwards, he may advantageously be provided with the first or second awareness mode in order to see his surroundings.

An embodiment of the first aspect is related to a medical viewing device, wherein the observation mode is registered to on one or more of:
- a position , in particular a position in the environment;
- an element identified in an image of the viewing device, in particular of an observation mode image; and
wherein the observation mode is only displayed if:
- the user looks, by the viewing device, in a direction in which the registered position and/or the registered image element can be captured by a camera of the viewing device; and/or
- the user looks, by the viewing device, in a direction, in which the observation mode view tied to a registered position intersects with a view of the first and/or the second awareness mode .

By registering the observation mode (view) to a position it is meant, that the observation mode does not move from this position. If the position is a position from the environment, this means, that the observation mode is registered to this position of the environment and does not move from this position. Such a position may also be referred to as a registered position. The environment of the user may be an operating room where the user is situated in front of a patient. A position in the environment may for example be right above the patient. So, if the user moves their head towards this position, and the observation mode is registered to this position, the user will be provided with the observation mode. In other words, if the user looks at this position they will see the observation mode. On the other hand, if a user looks away from that position, they will not be provided with the observation mode. A registration may be initiated with the push of a button, a voice command or a gesture, for example a hand gesture.

The observation mode may also or alternatively be registered to an identified element of an image of medical viewer. Insofar, an element may be recognized, for example by aforementioned image recognition techniques, and the observation mode may be registered to that image. Such an element may also be referred to as a registered element.

The observation mode may be displayed when the user directs their gaze or head towards the registered position in the environment, e.g. the position of a liver that is observed by a medical observation means.

In other words, the observation mode is registered to a spot in the environment and if the user directs their head at this spot, they will see the observation mode. Similarly, if the observation mode is registered to an element of an image, it may be displayed when the user directs his head at the image element, in particular if the user directs a camera mounted on or integrated in the medical viewing device, towards said element. In yet other words, the image element may be an element of the user's environment captured by a camera. If the user directs their head, and thus said camera, towards this element in the environment, the element will also be displayed in the awareness mode. If the observation mode is registered to such an element, it will be displayed at the position of the element.

An embodiment of the first aspect is related to a medical viewing device, wherein the first or the second awareness mode is only displayed if the user does not look, by the viewing device, into the direction of the registered position and/or the registered image element.

When a user looks at a registered position or a registered image element, the observation mode may be provided and not the first or second awareness mode. If however, the user does not look at a registered position or image element, the first or second awareness mode may be displayed. The transition may be seamless or may be a strict cut off. It is also possible, that both modes are shown in a transitional area. Furthermore, a hysteresis may be comprised so that the modes do not switch constantly in transitional areas, e.g. close to a registered position or image element. The observation mode can be prioritized over the first or second awareness mode in case the different modes overlap at a registered position or a registered image element. Such a configuration is in particular advantageous because a user is provided with the information of the observation mode when looking in respective direction and the user's field of vision is unobstructed by providing the first or second awareness view when turning their head.

An embodiment of the first aspect is related to a medical viewing device, wherein the first or the second awareness mode is displayed at display positions of the viewing device for which no observation mode view exists.

When a user looks away from a registered position or a registered image element, the first or second awareness mode may be provided and not the observation mode. If the observation mode is displayed on parts of the display, the first or second awareness mode may be displayed surrounding the observation mode. The transition may be seamless or may be a strict cut off. It is also possible, that both modes are shown simultaneously in a transitional area. Furthermore, a hysteresis may be comprised so that the modes do not switch constantly in transitional areas, e.g. close to a registered position or image element.

Such a configuration is in particular advantageous because the whole field of view of the medical viewing device may be utilized even if the observation mode does not require or fill the display completely.

An embodiment of the first aspect is related to a medical viewing device, wherein the viewing device is configured such that a peripheral vision of the user is unobstructed by the viewing device.

Such a configuration may in particular refer to an augmented reality device, where the peripheral view of the user is unobstructed by the device itself. Insofar, a user can use his actual field of view as much as possible and may additionally be provided with an observation mode or an awareness mode. An observation mode may for example be provided in the center of his field of view. The provided modes may in particular blend seamlessly with the user's peripheral vision.

A second aspect of the present disclosure is related to a method for operating a medical viewing device,
comprising the steps:
   - obtaining visual information about an environment from a medical observation means;
   - obtaining visual information about the environment from a camera;
   - displaying to the user the environment in two or more different modes ;
wherein in an observation mode the viewing device provides a view from the medical observation means; and
wherein in a first awareness mode, the viewing device provides a view from the camera.

With such a method, actions and procedures that require different fields of view can be performed during a medical procedure without folding the medical viewing device upwards or removing it. For example during surgery, a surgeon may require to both use a microscope and look at colleagues or at tools in his environment. With the method provided above, the different required fields of view may be navigated without the necessity of touching or moving the medical viewing device.

A medical observation device may be a digital camera, for example a camera of a microscope or mounted on a medical device. For example, an endoscope may be equipped with a camera, in particular a high-definition camera, to allow a surgeon to view internal organs and tissues. Thus, a medical observation device may be an external device. A camera may also be mounted on the medical viewing device in order to gather visual information from the environment surrounding the medical viewing device.

Obtaining visual information may comprise different actions of obtaining said information, for example fetching, receiving, determining. The medical viewing device may thus be configured to receive data, in particular image streams, from the medical observation device and/ or the camera. For example, a medical viewing device may receive data wirelessly from one or multiple medical observation devices or cameras. It is also possible, that a medical viewing device may receive such data via one or multiple digital or optical cables or wires. A view may also be considered an image stream, in particular a real time image stream, from a camera or a medical observation device of an environment. In other words, by obtaining visual information about an environment and providing a view from said camera or a medical observation device, the medical viewing device may provide the user with both a real-time image stream, or view, from the operating field through the medical observation device and also a real-time image stream, or view, from the room surrounding the patient, and possibly also the user, through the camera.

Displaying the environment to the user in two or more different modes may be carried out in different ways. For example, the medical viewing device may display only one mode at a time, for example spanning the whole screen of the medical viewing device and/ or the whole visual field of the user. Thus, a user has a complete and unobstructed view of the view provided by this, "current", mode. A user may switch in between modes, for example using gestures, eye movement, voice commands or other forms of physical interaction with the medical viewing device such as pressing a button. Displaying the environment to the user in two or more different modes may also be carried out by displaying said modes simultaneously. For example, the screen of the medical viewing device, or the visual field of the user, may be split in two or more areas so that each area may display the view provided by one mode. Said areas may also overlap or one area may be comprised in another. Insofar, a "window in window" representation is also possible, where one mode is displayed in a small area comprised in a larger area displaying another mode.

A method according to the second aspect of this disclosure may comprise steps that are described as functions of the medical viewing device of the first aspect of this disclosure.

### Brief description of the figures

Further advantages and features result from the following embodiments, some of which refer to the figures. The figures do not always show the embodiments to scale. The dimensions of the various features may be enlarged or reduced, in particular for clarity of description. For this purpose the figures are at least partially schematized.
Fig. 1 illustrates three different modes of a medical viewing device according to embodiments of this disclosure.
Fig. 2 illustrates a state machine for a medical viewing device according to embodiments of this disclosure.
Fig. 3 illustrates a mode of a medical viewing device according to embodiments of this disclosure
Fig. 4 illustrates microscope systems for embodiments of this disclosure.

In the following description reference is made to the accompanying figures which form part of the disclosure, and which illustrate specific aspects in which the present disclosure can be understood. Identical reference signs refer to identical or at least functionally or structurally similar features.

In general, a disclosure of a described method also applies to a corresponding device (or apparatus) for carrying out the method or a corresponding system comprising one or more devices and vice versa. For example, if a specific method step is described, a corresponding device may include a feature to perform the described method step, even if that feature is not explicitly described or represented in the figure. On the other hand, if, for example, a specific device is described on the basis of functional units, a corresponding method may include one or more steps to perform the described functionality, even if such steps are not explicitly described or represented in the figures. Similarly, a system can be provided with corresponding device features or with features to perform a particular method step. The features of the various exemplary aspects and embodiments described above or below may be combined unless expressly stated otherwise.

### Detailed description

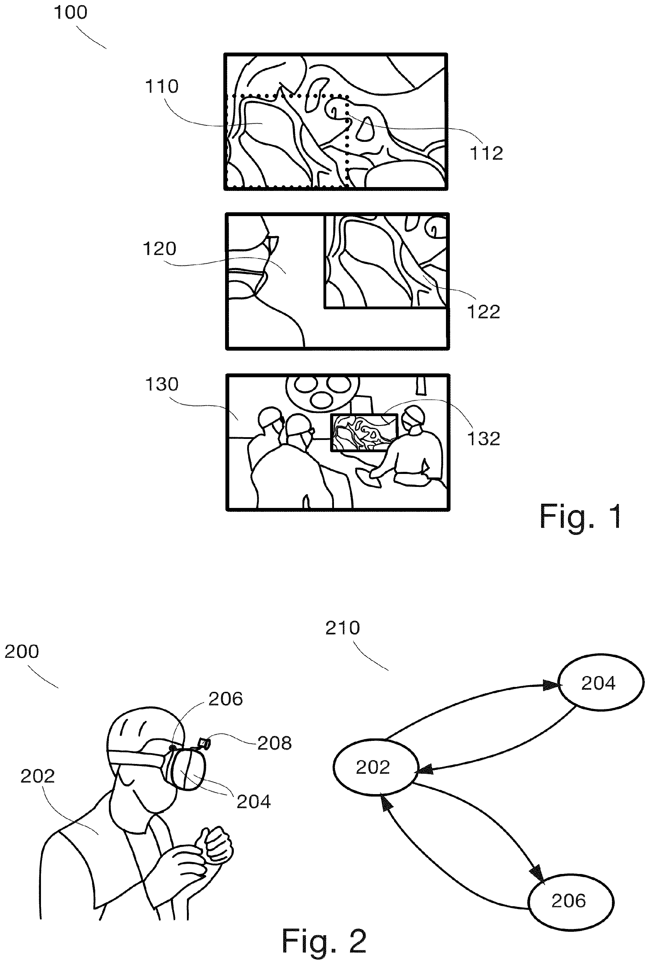

In Fig.1 a modes-set-up 100 of three different modes of a medical viewing device according to embodiments of this disclosure are illustrated. Fig. 1 illustrates an observation mode 110, a first awareness mode 120 and a second awareness mode 130. In the progression from the observation mode 110 to the second awareness mode 130 it can be seen that each progressing mode 110, 120 and 130 shows a view that is more "zoomed out" than the mode before. The observation mode 110 shows a very detailed view of the operating field, for example an intestine of a patient in a close up. The observation model 10 can also be configured to provide a magnified view 112 of the view obtained from the medical observation means.

The first awareness mode 120 shows view that is closer to the natural view of the user of the medical viewing device. Insofar, the view of the first awareness mode 120 may be obtained by a camera mounted on the medical viewing device or integrated in the medical viewing device. As can be seen, the first awareness mode 120 also shows a part of the observation mode view 112. For example, a screen may be set up in the operating room showing the view 122 obtained by the medical observation means. Using the first awareness mode 120, the user may be provided with a more natural view of their surroundings, including objects, like said screen showing view 122, and also colleagues. in the operating room.

The second awareness mode 130 shows a more zoomed out view, i.e. a wide-field view, compared to the two previous modes 110 and 130. The second awareness mode 130 shows a view from a camera, for example be an external camera, that may not be mounted on the medical viewing device but is instead mounted to a wall or the ceiling of the operating room. As can be seen, the second awareness mode 130 also comprises (on an observed screen 132) the observation mode view 110. As in the first awareness mode 120, a screen may be set up in the operating room showing the view obtained by the medical observation means.

The surgeon seen in the second awareness mode 130 evidently cannot use their hands to switch modes as they are performing a medical procedure. Furthermore, the screen showing the observation view 122 may be positioned behind this surgeon, so that they have to turn their neck to see the view 122.

The medical viewing device can provide this surgeon with all three modes 110, 120 and 130 without the necessity of moving their head towards the screen. Furthermore, the surgeon may switch between modes using head-based gestures, so that their hands can remain with the medical procedure they are carrying out.

A head-based gesture may be recognized or identified based on one or more of the aforementioned types of information, in particular on a position-based information, an information in an image or an information from an external sensor. For example, a head-based gesture may be recognized based on the orientation and movement of the medical viewing device in three-dimensional space, such as pitch, yaw, and roll. Insofar, a head-based gesture may for example be a surgeon moving their head away from the patient. In one example, a switch between modes may be carried out where the observation mode 110 was provided as long as the surgeon's head was directed towards the patient and the first or second awareness 120, 130 mode may be provided as soon as the surgeon turned their head away from the patient. In one example, a switch between the first and second awareness mode 120, 130 may be carried out based on how far a surgeon has turned their head away from the patient.

Fig. 2 illustrates a surgeon wearing a medical viewing device 200 according to an embodiment of this disclosure. Furthermore, an exemplary state machine 200 for operating a medical viewing device according to embodiments of this disclosure is illustrated.

On the left side of Fig. 2, a surgeon 202 wearing the medical viewing device 200 is depicted. The medical viewing device normally provides a medical mode view, e.g. an image of a microscope, an endoscope, or alike. The medical viewing device 200 can be worn, e.g. with a rubber strap, around the surgeon's head. The medical viewing device may comprise a joint 206 by which the display 204 of the viewing device can be positioned upwards, such that the surgeon can look directly at the environment. However to position the display upwards, someone, e.g. the surgeon, needs to touch the viewing device. During surgery, natural view information or other awareness information should be provided without mechanically re-positioning the device 204. This is done by providing different view modes to the surgeon via the display(s) 204 of the medical viewing device 200.

The illustrated medical viewing device 200 has a display 203, which comprises a left and a right subdisplay, in order to display 3D information. Furthermore, the medical viewing device 200 comprises a camera 208. The camera observes the environment from the same perspective as the surgeon and can therefore provide to the surgeon a natural view image in a first awareness mode. The device 200 further comprises a wireless interface to receive further camera information that can be used for a second awareness mode. Also the information of the medical observation means (not depicted) can be provided to the medical viewing device 200 via this interface.

On the right side of Fig. 2, a state machine for operating the different modes of the medical viewing device 200 is illustrated. Each knot represents a mode. For example, the knot on the left may represent the activation of the first awareness mode 210. From this knot, when a mode switching occurs, it is possible to switch to the activation of the second awareness mode 230 and back. If a user is in a first awareness mode 210 but requires a wider field of view or a different angle, she/he may switch to the second awareness mode 230. If the user has been provided with the visual information they required, a switch back to the first observation mode 210 is possible.

It is also possible to switch from the first awareness mode 210 to the observation mode 220. If for example, a user is in the first awareness mode 210 but requires a view that is much more in detail of the operating field, they may want to switch to the observation mode 220. In the observation mode 220, the surgeon may be provided with visual information obtained by the medical observation means, for example a microscope. Once the user has obtained all the visual information required from the medical observation means, a switch back to the first awareness mode 210 is possible.

A switching between modes may be based on one or more of: a position-based information of the viewing device, an information in an image obtained by the viewing device and/or an information from an external sensor that observes a position and/or a motion of the medical viewing device. While not shown in Fig. 2, a switch from the observation mode 220 directly to the second awareness mode 230 is generally also possible if required.

In a state machine of another embodiment of the medical viewing device 200, switching from every mode to every other mode is possible. This provides more freedom for the user to switch between the different modes. However, in this case the user has to control the device with at least two switching commands, one for each potential mode transition.

In another state machine of a further embodiment of this disclosure, the user has optional modes that she/he can configure before operation and/or during operation. For example, the user can add additional awareness modes, based on further camera perspectives to the state machine. Additionally or alternatively, one or more additionally observation modes may be added. For example, a certain resolution if a microscope can be determined as an additional observation mode, while the normal observation mode may operate with a different resolution.

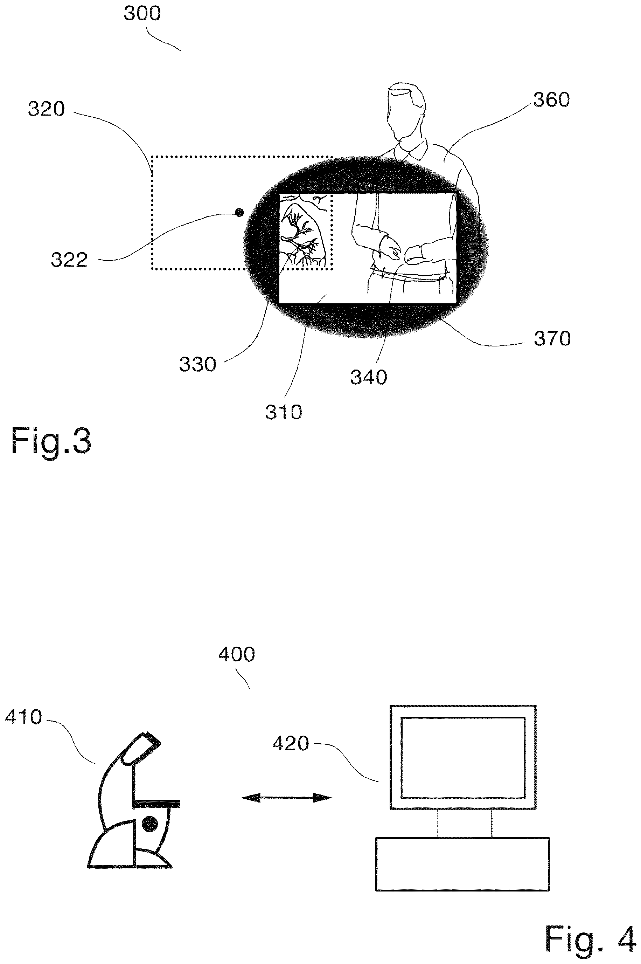

In Fig. 3 a mode 300 of a medical viewing device according to embodiments of this disclosure is illustrated. In particular, an intersection of different modes is illustrated. Insofar, both a first awareness mode 310 and also an observation mode 320 are illustrated. However, the view of the user in the first awareness mode 310 is directed towards a person 360 and only partially intersects with the observation mode 320. Insofar, the user is not provided with the whole view of the observation mode 320 but instead only with the view in the intersecting area 330 of the observation mode 320. The intersecting area 330 of the observation mode 320 is that part of the observation mode 320, that intersects or overlaps with the first awareness mode 310. At the intersecting area 330, the view 310 of the first awareness mode 310 is not shown but rather the view of the observation mode. Within the first awareness mode 310, the view is only shown in those areas that do not overlap or intersect with the observation mode 320. The black ellipsoid 370 illustrates the surroundings of the visual display of the medical viewing device.

The observation mode 320 may for example be registered to a position 322. By registering the observation mode 320 to a position is meant, that the observation mode 320 does not move from this position. If the user moves their head towards a position, and the observation mode 320 is registered to this position, the user will be provided with the observation mode 320 and the complete view 322 of the observation mode 320. On the other hand, if a user looks away from that position, they will not be provided with the observation mode 320 or they will at least not see the complete view 322. A registration may for example be initiated with the push of a button, a voice command or a gesture, for example a hand gesture.

When, as shown in Fig. 3, a user does not look directly at the observation mode 320 but slightly to the side of the observation mode 320, the view of the first awareness mode 310 may be provided in those areas that do not intersect with the observation mode 320. As shown in fig. 3, the view 330 of the observation mode 320 may then be displayed on parts of the field of vision of the user and the view 310 of the first awareness mode 310 may be displayed surrounding the view 330 of the observation mode 320. Insofar, both modes 310 and 320 are shown simultaneously in a transitional area. Advantageously, a hysteresis may be comprised so that the modes 310 and 320 do not switch constantly in transitional areas. Such a configuration is in particular advantageous because the whole field of view of the medical viewing device may be utilized even if the observation mode 320 does not require or fill the display completely.

Furthermore, it can be seen in Fig. 3, that the person 360 outside of the first awareness mode 310 comprises the same distance from the user and the same proportions as the person 340 as seen within the first awareness mode 310. Thus, the first awareness mode 310 must be relatively similar to the natural view and natural field of vision of the user.

Some embodiments relate to a microscope comprising a system as described in connection with one or more of the Figs. 1 to 3. Alternatively, a microscope may be part of or connected to a system as described in connection with one or more of the Figs. 1 to 3. Fig. 4 shows a schematic illustration of a system 400 configured to perform a method described herein. The system 400 comprises a microscope 410 and a computer system 420. The microscope 410 is configured to take images and is connected to the computer system 420. The computer system 420 is configured to execute at least a part of a method described herein. The computer system 420 may be configured to execute a machine learning algorithm. The computer system 420 and microscope 410 may be separate entities but can also be integrated together in one common housing. The computer system 420 may be part of a central processing system of the microscope 410 and/or the computer system 420 may be part of a subcomponent of the microscope 410, such as a sensor, an actor, a camera or an illumination unit, etc. of the microscope 410.

The computer system 420 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 420 may comprise any circuit or combination of circuits. In one embodiment, the computer system 420 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 420 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 420 may include one or more storage devices, which may include one or more memory elements suitable to the particular application, such as a main memory in the form of random-access memory (RAM), one or more hard drives, and/or one or more drives that handle removable media such as compact disks (CD), flash memory cards, digital video disk (DVD), and the like. The computer system 420 may also include a display device, one or more speakers, and a keyboard and/or controller, which can include a mouse, trackball, touch screen, voice-recognition device, or any other device that permits a system user to input information into and receive information from the computer system 420.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Embodiments may be based on using a machine-learning model or machine-learning algorithm. Machine learning may refer to algorithms and statistical models that computer systems may use to perform a specific task without using explicit instructions, instead relying on models and inference. For example, in machine-learning, instead of a rule-based transformation of data, a transformation of data may be used, that is inferred from an analysis of historical and/or training data. For example, the content of images may be analyzed using a machine-learning model or using a machine-learning algorithm. In order for the machine-learning model to analyze the content of an image, the machine-learning model may be trained using training images as input and training content information as output. By training the machine-learning model with a large number of training images and/or training sequences (e.g. words or sentences) and associated training content information (e.g. labels or annotations), the machine-learning model "learns" to recognize the content of the images, so the content of images that are not included in the training data can be recognized using the machine-learning model. The same principle may be used for other kinds of sensor data as well: By training a machine-learning model using training sensor data and a desired output, the machine-learning model "learns" a transformation between the sensor data and the output, which can be used to provide an output based on non-training sensor data provided to the machine-learning model. The provided data (e.g. sensor data, meta data and/or image data) may be preprocessed to obtain a feature vector, which is used as input to the machine-learning model.

Machine-learning models may be trained using training input data. The examples specified above use a training method called "supervised learning". In supervised learning, the machine-learning model is trained using a plurality of training samples, wherein each sample may comprise a plurality of input data values, and a plurality of desired output values, i.e. each training sample is associated with a desired output value. By specifying both training samples and desired output values, the machine-learning model "learns" which output value to provide based on an input sample that is similar to the samples provided during the training. Apart from supervised learning, semi-supervised learning may be used. In semi-supervised learning, some of the training samples lack a corresponding desired output value. Supervised learning may be based on a supervised learning algorithm (e.g. a classification algorithm, a regression algorithm or a similarity learning algorithm. Classification algorithms may be used when the outputs are restricted to a limited set of values (categorical variables), i.e. the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are. Apart from supervised or semi-supervised learning, unsupervised learning may be used to train the machine-learning model. In unsupervised learning, (only) input data might be supplied, and an unsupervised learning algorithm may be used to find structure in the input data (e.g. by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters.

Reinforcement learning is a third group of machine-learning algorithms. In other words, reinforcement learning may be used to train the machine-learning model. In reinforcement learning, one or more software actors (called "software agents") are trained to take actions in an environment. Based on the taken actions, a reward is calculated. Reinforcement learning is based on training the one or more software agents to choose the actions such, that the cumulative reward is increased, leading to software agents that become better at the task they are given (as evidenced by increasing rewards).

Furthermore, some techniques may be applied to some of the machine-learning algorithms. For example, feature learning may be used. In other words, the machine-learning model may at least partially be trained using feature learning, and/or the machine-learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e. outlier detection) may be used, which is aimed at providing an identification of input values that raise suspicions by differing significantly from the majority of input or training data. In other words, the machine-learning model may at least partially be trained using anomaly detection, and/or the machine-learning algorithm may comprise an anomaly detection component.

In some examples, the machine-learning algorithm may use a decision tree as a predictive model. In other words, the machine-learning model may be based on a decision tree. In a decision tree, observations about an item (e.g. a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree.

Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may e.g. be used to store, manipulate or apply the knowledge.

Machine-learning algorithms are usually based on a machine-learning model. In other words, the term "machine-learning algorithm" may denote a set of instructions that may be used to create, train or use a machine-learning model. The term "machine-learning model" may denote a data structure and/or set of rules that represents the learned knowledge (e.g. based on the training performed by the machine-learning algorithm). In embodiments, the usage of a machine-learning algorithm may imply the usage of an underlying machine-learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g. of the sum of its inputs). The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e. to achieve a desired output for a given input.

Alternatively, the machine-learning model may be a support vector machine, a random forest model or a gradient boosting model. Support vector machines (i.e. support vector networks) are supervised learning models with associated learning algorithms that may be used to analyze data (e.g. in classification or regression analysis). Support vector machines may be trained by providing an input with a plurality of training input values that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection.

### List of reference signs

- 100: modes set up
- 110: observation mode
- 112: part of observation mode image
- 120: first awareness mode
- 122: view of medical observation means
- 130: second awareness mode
- 132: screen
- 200: surgeon with medical viewing device
- 202: surgeon
- 204: displays
- 206: joint
- 208: camera
- 300: awareness mode with observation mode at registered position
- 310: first awareness mode
- 320: observation mode
- 322: position to register observation mode
- 330: intersection of two views
- 340: naturally viewed person
- 360: person
- 370: display surroundings of device
- 400: microscope system
- 410: microscope
- 420: computer system

## Claims

1. Medical viewing device,
configured to:
- be worn by a human user, in particular on a user's head;
- obtain visual information about an environment from a medical observation means;
- obtain visual information about the environment from a camera;
- display to the user the environment in two or more different modes (110, 120, 130);
wherein in an observation mode the viewing device provides a view from the medical observation means; and
wherein in a first awareness mode, the viewing device provides a view from the camera.

2. The medical viewing device according to the preceding claim,
comprising one or more cameras.

3. The medical viewing device according to one of the preceding claims, configured to:
- obtain information from one or more external cameras.

4. The medical viewing device according to one of the preceding claims, wherein in the first awareness mode the viewing device provides a view which is less magnified than in the observation mode.

5. The medical viewing device according to the preceding claim,
wherein in a second awareness mode, the viewing device provides a view which is less magnified than in the first awareness mode.

6. The medical viewing device according to one of the preceding claims, configured to:
- switch between modes based on one or more of:
-- a position-based information of the viewing device;
-- an information in an image obtained by the viewing device;
-- an information from an external sensor that observes a position and/or a motion of the medical viewing device.

7. The medical viewing device according to one of the preceding claims, configured to:
- switch between modes based on a gesture by the user, in particular by:
- - a head-based gesture;
- - a hand-based gesture.

8. The medical viewing device according to one of the preceding claims, configured to:
- switch between modes based on a gesture by the user, such that the viewing device switches to the observation mode if the head of the user moves slower than a pre-defined threshold.

9. The medical viewing device according to one of the preceding claims, configured to:
- switch between modes based on a gesture by the user, such that the viewing device switches to the first or the second awareness mode if the head of the user moves faster than a pre-defined threshold.

10. The medical viewing device according to one of the preceding claims, configured to:
- switch between modes based on a gesture by the user, such that the viewing device switches to the observation mode or to the first or the second awareness mode if the user performs a gesture based on tilting the user's head.

11. The medical viewing device according to one of the preceding claims,
wherein the observation mode (520) is registered to on one or more of:
- a position (522), in particular a position in the environment;
- an element identified in an image of the viewing device, in particular of an observation mode image; and
wherein the observation mode (520) is only displayed if:
- the user looks, by the viewing device, in a direction in which the registered position (522) and/or the registered image element can be captured by a camera of the viewing device; and/or
- the user looks, by the viewing device, in a direction, in which the observation mode view tied to a registered position (522) intersects with a view of the first and/or the second awareness mode (540).

12. The medical viewing device according to the preceding claim,
wherein the first or the second awareness mode (540) is only displayed if the user does not look, by the viewing device, into the direction of the registered position and/or the registered image element.

13. The medical viewing device according one of the two preceding claims, wherein the first or the second awareness mode (540) is displayed at display positions of the viewing device for which no observation mode view exists.

14. The medical viewing device according to one of the preceding claims, wherein the viewing device is configured such that a peripheral vision of the user is unobstructed by the viewing device.

15. A method for operating a medical viewing device,
comprising the steps:
- obtaining visual information about an environment from a medical observation means;
- obtaining visual information about the environment from a camera;
- displaying to the user the environment in two or more different modes (110, 120, 130);
wherein in an observation mode the viewing device provides a view from the medical observation means; and
wherein in a first awareness mode, the viewing device provides a view from the camera.
